# EUROPÄISCHE PATENTSCHRIFT

(11) **EP 2 236 167 B1**
(45) Veröffentlichungstag und Bekanntmachung des Hinweises auf die Patenterteilung: **12.10.2011**
(21) Anmeldenummer: 09156682.8
(22) Anmeldetag: 30.03.2009
(51) Int. Cl.: A61M 16/16, A61M 5/168, A61M 16/00

(54) **Befeuchtungsvorrichtung für Atemgase**
Humidifier for breathing gases
Dispositif d'humidification pour gaz respiratoires

(43) Veröffentlichungstag der Anmeldung: 06.10.2010
(73) Patentinhaber: Dräger Medical GmbH, 23558 Lübeck (DE)
(72) Erfinder: Rossen, Thomas, 23560, Lübeck (DE); Brieger, Kai, 21033 Hamburg (DE); Tappehorn, Ludger, 23560, Lübeck (DE); Kämer, Markus, 23552, Lübeck (DE)
(74) Vertreter: UEXKÜLL & STOLBERG

(56) Entgegenhaltungen:
- EP-A1- 0 589 429
- WO-A1-2008/148154
- DE-A1- 2 644 584
- US-A- 4 014 010
- US-A1- 2003 098 022

## Beschreibung

Die Erfindung betrifft eine Befeuchtungsvorrichtung für Atemgase gemäß Anspruch 1 und ein Verfahren zum Betreiben einer Befeuchtungsvorrichtung für Atemgase gemäß Anspruch 11.

Eine künstliche Beatmung von Patienten erfolgt nach dem Stand der Technik mit Hilfe von Beatmungsgeräten, die ein Atemgas im Überdruckverfahren zu dem Patienten leiten. Das Atemgas wird entweder von einem Ventilator aus der Umgebungsluft angesaugt oder gelangt aus einer zentralen Gasversorgung in eine Befeuchtungsvorrichtung. Befeuchtungsvorrichtungen werden während der Beatmung eines Patienten verwendet, um dem dem Patienten zugeleiteten Atemgas während jeder Einatmungsphase Wasserdampf zuzufügen, damit das aus der Umgebung angesaugte oder aus der zentralen Gasversorgung eingeleitete, im Wesentlichen trockene Atemgas temperiert oder auf ein Maß an oder nahe einer vollständigen Feuchtigkeitssättigung zu befeuchten.

Eine solche Befeuchtungsvorrichtung ist aus der US 6,272,933 B1 bekannt. Die Befeuchtungsvorrichtung enthält eine wieder befüllbare Verdunsterkammer, die dazu bestimmt ist, zu verdunstendes Wasser aufzunehmen, wobei die Verdunsterkammer die in Kontakt mit einer Heizvorrichtung innerhalb eines Gehäuses angeordnet ist. Die Verdunsterkammer enthält eine Wasserzuführung, in die Wasser aufgrund von Schwerkraft aus einem Wasserreservoir durch eine Öffnung in die Verdunsterkammer gelangt. Ferner gelangt durch eine weitere Öffnung Atemgas in die Verdunsterkammer, welches in dieser erhitzt und befeuchtet werden soll. Bei einer oft tagelangen Beatmung der Patienten mit einer entsprechenden Anfeuchtung des Atemgases kann der Wasservorrat in der Verdunsterkammer schnell aufgebraucht werden. Ein erforderliches Nachfüllen des Wassers in der Verdunsterkammer erfolgt nach dem Stand der Technik kontinuierlich durch eine für diesen Zweck speziell entwickelte Ventiltechnik, wie sie beispielsweise in der DE 693 23 518 T2 beschrieben ist. Ein automatisches Befüllen der Verdunsterkammer mit Wasser ist jedoch aufgrund eines begrenzten Volumens des Wasserreservoirs limitiert, wodurch eine weitere Anfeuchtung der Atemgase bei einem aufgebrauchten Wasserreservoir nicht mehr erfolgen kann.

Eine Lösung dieses Problems wird in der US 6,272,933 B1 beschrieben, wonach die Daten eines am Atemgasauslasskanal der Verdunsterkammer angebrachten Temperatursensors und eines Messsignals des Atemgasflows durch die Kammern ausgewertet werden. Ein Algorithmus ermittelt daraus einen Zustand der Verdunsterkammer ohne Wasser. Nachteilig an dieser Lösung ist jedoch, dass eine Alarmierung nur zeitversetzt erfolgen kann, nämlich nachdem die Auswertung abgeschlossen worden ist, mit der Folge einer möglichen Beeinflussung eines Therapieverlaufs.

Die Erfindung bezweckt, den oben genannten Nachteil zu beseitigen. Diese Aufgabe wird durch und eine Vorrichtung und ein Verfahren mit den Merkmalen der unabhängigen Ansprüche gelöst. In den davon abhängigen Ansprüchen sind vorteilhafte Ausgestaltungen und Weiterbildungen der Erfindung angegeben.

Eine Befeuchtungsvorrichtung für Atemgase nach der Erfindung ist dadurch gekennzeichnet, dass die Befeuchtungsvorrichtung ein Gehäuse mit einer Ausnehmung, einer Heizvorrichtung, einer Verdunsterkammer mit einer Wasserzuführung, einem Atemgaszuführungskanal, einem Atemgasauslasskanal und wenigstens einem Schwimmerelement und wenigstem einem Füllstandsdetektor umfasst, wobei die Verdunsterkammer in die Ausnehmung wenigstens teilweise einbringbar ist, in Kontakt mit der Heizvorrichtung zum Erwärmen von Wasser steht und ausgeführt ist, Wasser aus einem Wasserreservoir durch die Wasserzuführung aufzunehmen, wobei das wenigstens eine Schwimmerelement auf dem Wasser schwimmend beweglich geführt ist und ausgebildet ist, die Wasserzuführung oberhalb eines Wasserstandssollwertes in der Verdunsterkammer zu verschließen und der wenigstens eine Füllstandsdetektor ausgebildet ist, in Abhängigkeit von der Position des wenigstens einen Schwimmerelements ein Absinken des Wassers von dem Wasserstandssollwert zu erkennen.

In vorteilhafter Weise kann die erfindungsgemäße Befeuchtungsvorrichtung für Atemgase einen Wassermangel in der Verdunsterkammer frühzeitig signalisieren, so dass ein Wasserreservoir zur Befüllung der Verdunsterkammer ausgetauscht oder aufgefüllt werden kann, bevor es zu einer Unterfeuchtung des an den Patienten gelangenden Atemgases kommen kann.

Vorzugsweise wird entsprechend einer Ausführungsform der Erfindung der Füllstandsdetektor als eine auf den Wasserstandssollwert angepasste optische Sensoranordnung ausgeführt, die aus einem Lichtsender und einem Lichtempfänger besteht. Die Verdunsterkammer ist einstückig aus einem lichtdurchlässigen Material, vorzugsweise aus einem PU, ausgebildet. Somit kann die optische Sensoranordnung auch in vorteilhafter Weise außerhalb der Verdunsterkammer und innerhalb des Gehäuses der Befeuchtungsvorrichtung angeordnet und so eingestellt sein, dass ein von dem Lichtsender in die Verdunsterkammer ausgesendeter Lichtstrahl von dem Schwimmerelement unterbrochen wird, wenn die Höhe des Wassers in der Verdunsterkammer dem Wasserstandssollwert entspricht. Der Wasserstandssollwert entspricht wiederum einer optimalen Höhe des Wasserstandes im normalen Betriebsmodus der Befeuchtungsvorrichtung.

Die Verdunsterkammer weist in dem Bodenbereich eine Kontaktfläche zur Übertragung von Wärme von der Heizvorrichtung auf das Wasser und damit für die Verdunstung des Wassers in der Verdunsterkammer auf. Das Schwimmerelement regelt den Wasserstand in der Verdunsterkammer durch Abdichten der Öffnung der Wasserzuführung bei einer Überschreitung des Wasserstandes über dem Wasserstandsollwert. Die Wasserzuführung ist mit einem separaten Wasserreservoir verbunden. Wenn Wasser benötigt wird, fällt der Wasserstand in der Verdunsterkammer, wobei die Wasserzuführung geöffnet wird. Damit kann zusätzliches Wasser aus dem Wasserreservoir in die Verdunsterkammer nachfließen. Zur zeitnahen Erkennung einer leere Verdunsterkammer, ist eine Ausführungsform von Bedeutung, die dadurch gekennzeichnet ist, dass Mittel vorgesehen sind aus dem Signal des Füllstandsdetektors und einem Signal eines Flowsensors eine Restzeit des verbleibenden Wassers in der Verdunsterkammer zu ermitteln und ein entsprechendes Ausgangssignal zur Anzeige und Alarmgabe zu erzeugen. Damit kann der Anwender zeitnah über eine Restzeit des in der Verdunsterkammer verbleibenden Wasser informiert werden und innerhalb dieser Zeit zur Gewährleistung eines stabiles Beatmungsbetriebes das Wasserreservoir austauschen bzw. auffüllen. Der Flowsensor ist dabei vorzugsweise an dem Atemgasauslasskanal angeordneten. Alternativ hierzu kann der Flowsensor in der Nähe des Ventilators angeordnet sein, wobei das Signal des Flowsensor über eine Datenschnittstelle von dem Ventilator der Befeuchtungsvorrichtung zur Verfügung gestellt wird.

Im Hinblick auf Störungen an dem Schwimmerelementverschluss ist eine Ausführung von Bedeutung, die dadurch gekennzeichnet ist, dass ein zweites Schwimmerelement und ein zweiter Füllstandsdetektor in der Verdunsterkammer vorgesehen sind, wobei das zweite Schwimmerelement in der Verdunsterkammer über die Höhe des Wasserstand-Sollwertes hinaus bewegbar ist und der zweite Füllstandsdetektor vorgesehen ist, eine Position des zweiten Schwimmerelements oberhalb des Wasserstand-Sollwertes zu erkennen. In vorteilhafter Weise sind ferner Mittel vorgesehen, aus dem Signal des zweiten Füllstandsdetektors ein entsprechendes Ausgangssignal zur Anzeige und Alarmgabe zu erzeugen.

Eine weitere Ausführungsform ist dadurch gekennzeichnet, dass das zweite Schwimmerelement das erste Schwimmerelement konzentrisch umgibt und in vorteilhafter Weise das zweite Schwimmerelement auf dem ersten Schwimmerelement aufliegt. Somit kann in vorteilhafter Weise in Abhängigkeit von der Position des zweiten Schwimmerelements ein Ansteigen des Wassers innerhalb der Verdunsterkammer oberhalb des Wasserstand-Sollwertes von dem zweiten Füllstandsdetektor erkannt werden.

Sowohl der erste als auch der zweite Füllstandsdetektor kann als eine magnetische Sensoranordnung ausgeführt sein, die aus einem Hallsensor und ein an dem jeweiligen Schwimmerelement einstückig angeformten Magnet besteht.

Ferner ist die Erfindung durch das erfindungsgemäße Verfahren zum Betreiben einer Befeuchtungsvorrichtung für Atemgase gekennzeichnet, welches die folgenden Verfahrensschritte aufweist: a) Zuführung von Wasser in einem Wasserreservoir in eine Verdunsterkammer, b) Sperren der Wasserzuführung an der Verdunsterkammer durch ein Schwimmerelement bei Überschreitung eines Wasserstand-Sollwertes in der Verdunsterkammer, c) Erwärmung des Wassers in der Verdunsterkammer durch eine Heizvorrichtung und d) Detektierung eines Absinken des Wassers von dem Wasserstand-Sollwert mit einem Füllstandsdetektor in Abhängigkeit von der Position des Schwimmerelements.

In einem weiteren Verfahrensschritt können ein Signal des Füllstandsdektors und ein Signal eines Flowsensors erfasst werden, aus welchem in vorteilhafter Weise nach dem Verfahrensschritt d) eine Restzeit des verbleibenden Wassers in der Verdunsterkammer bestimmt wird.

In einem wiederum weiteren Verfahrensschritt wird in Abhängigkeit der Restzeit des verbleibenden Wassers in der Verdunsterkammer ein entsprechendes Ausgangssignal zur Anzeige und Alarmgabe erzeugt.

Die vorliegende Erfindung wird mit Bezug auf die beigefügten Zeichnungen detaillierter erläutert, wobei gleiche Bezugszeichen gleiche Elemente bezeichnen.

In der Zeichnung gilt:
- Figur 1: ist eine schematische Darstellung eines Atemsystems mit der erfindungsgemäßen Befeuchtungsvorrichtung,
- Figur 2: ist ein Teilausschnitt einer Schnittdarstellung der Verdunsterkammer der erfindungsgemäßen Befeuchtungsvorrichtung
- Figur 3: ist ein Teilausschnitt einer Schnittdarstellung der Verdunsterkammer der erfindungsgemäßen Befeuchtungsvorrichtung mit einem ersten und einem zweiten Füllstandsdetektor,
- Figur 4: ist eine schematische Darstellung der Verdunsterkammer mit Wasserreservoir im Zustand "normaler Betrieb".
- Figur 5: ist eine schematische Darstellung der Verdunsterkammer mit Wasserreservoir im Zustand "Wasser leer" und
- Figur 6: ist eine schematische Darstellung der Verdunsterkammer mit Wasserreservoir im Zustand "fehlerhaften Dosierventil".

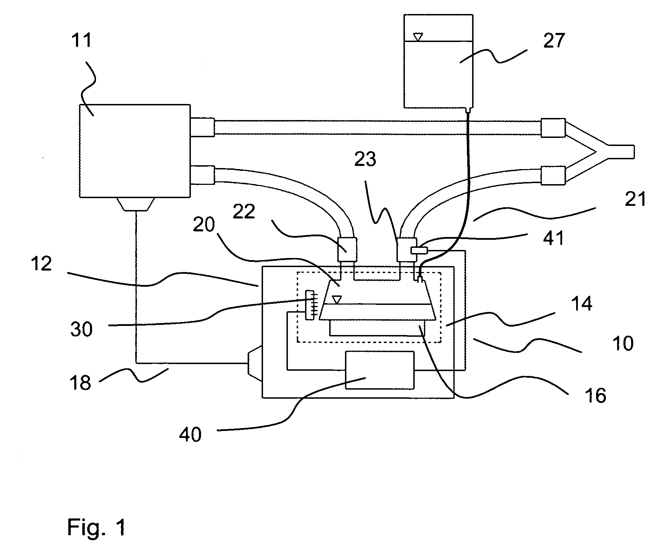

In Figur 1 ist ein Atemsystem mit einer erfindungsgemäßen Befeuchtungsvorrichtung 10 für eine Anreicherung der Atemgase mit Feuchtigkeit dargestellt. Das Atemsystem enthält einen Ventilator 11 zur Bereitstellung von Atemgas, welches über einen Inspirationsschlauch durch einen Atemgaszuführungskanal 22 in eine Verdunsterkammer 20 geleitet wird. In der Verdunsterkammer 20 wird über die Oberfläche einer Wassermenge das Atemgas erwärmt und angefeuchtet. Das Atemgas gelangt durch einen Atemgasauslasskanal 23 der Verdunsterkammer 20 über einen Schlauch an den zu beatmenden Patienten. Ein Exspirationsschlauch führt das ausgeatmete Atemgas an den Ventilator 11 zurück. Die Befeuchtungsvorrichtung 10 umfasst ein Gehäuse 12 mit einer Ausnehmung 14, in der die Verdunsterkammer 20 einbringbar ist. Die Verdunsterkammer 20 steht in der Ausnehmung 14 in Kontakt mit einer Heizvorrichtung 16 zur Erwärmung von Wasser der Verdunsterkammer 20. Über eine Wasserzuführung 21 gelangt Wasser eines Wasserreservoirs 27 in die Verdunsterkammer 20. Das Wasser fließt von dem oberhalb der Verdunsterkammer 20 angeordneten Wasserreservoir 27 aufgrund der Schwerkraft durch die Wasserzuführung 21 in die Verdunsterkammer 20.

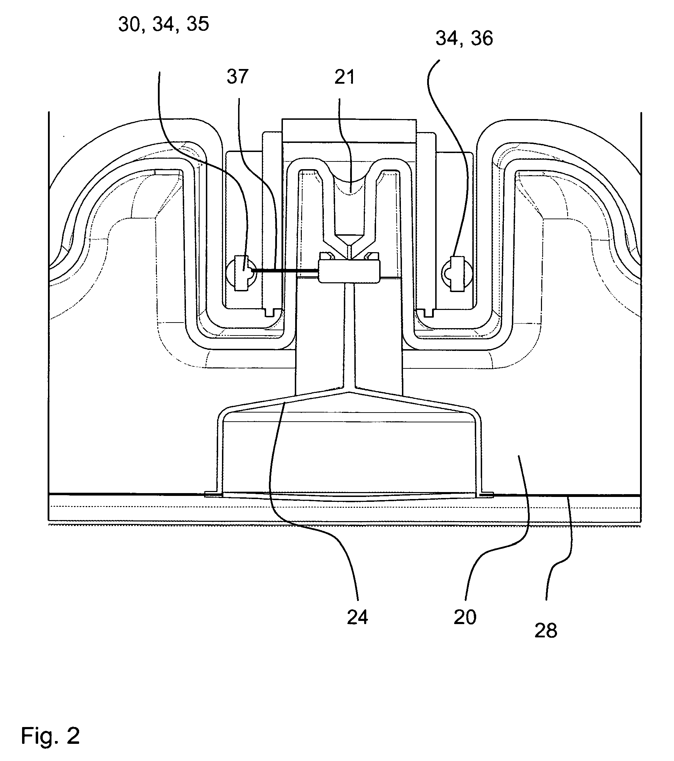

Figur 2 zeigt ein Teilausschnitt einer Schnittdarstellung der Verdunsterkammer 20 der erfindungsgemäßen Befeuchtungsvorrichtung 10. Durch die Wasserzuführung 21 gelangt Wasser eines nicht dargestellten Wasserreservoirs in die Verdunsterkammer 20. Ein Schwimmerelement 24, welches auf der Wasseroberfläche schwimmend beweglich geführt ist, ist dabei so ausgebildet, die Wasserzuführung 21 bei Überschreitung eines Wasserstand-Sollwertes 28, zu verschließen. Der Wasserstand-Sollwertes 28 entspricht der Höhe des Wassers in der Verdunsterkammer eines normalen Verdunsterbetriebes. Das Schwimmerelement 24 und die Wasserzuführung 21 sind als ein sogenanntes Schwimmerelementventil ausgeführt, wobei das Schwimmerelement 24 und die Wasserzuführung 21 im gegenseitigen Zusammenwirken den Wasserstand in der Verdunsterkammer durch Abdichten der Öffnung der Wasserzuführung 21 regeln, insbesondere wenn der Wasserstand-Sollwert 28 erreicht ist. Falls Wasser in der Verdunsterkammer 20 benötigt wird, fällt der Wasserstand, wobei die Öffnung der Wasserzuführung 21 vergrößert wird und damit zusätzliches Wasser aus dem Wasserreservoir 27 in die Verdunsterkammer 20 fließen kann.

Ferner ist in der Figur 2 ein Füllstandsdetektor 30 gezeigt. Der Füllstandsdetektor 30 ist als eine auf den Wasserstand-Sollwert 28 angepasste optische Sensoranordnung 34 ausgeführt. Die optische Sensoranordnung 34 besteht aus einem Lichtsender 35 und einem Lichtempfänger 36. Die Verdunsterkammer 20 ist einstückig vorzugsweise aus einem lichtdurchlässigen Material ausgeführt. Die optische Sensoranordnung 34 ist innerhalb des Gehäuses 12 der Befeuchtungsvorrichtung 10 vorgesehen. Der Lichtsender 35 und der Lichtempfänger 36 sind dabei so an der Verdunsterkammer 20 angeordnet, dass ein von dem Lichtsender 35 in die Verdunsterkammer 20 ausgesendeter Lichtstrahl 37 von dem Schwimmerelement 24 unterbrochen wird, wenn die Höhe des Wassers der Verdunsterkammer 20 dem Wasserstand-Sollwert 28 entspricht. Der Lichtstrahl 37 gelangt somit in der Höhe des Schwimmerelements 24, die dem Wasserstand-Sollwert 28 entspricht, nicht an den Lichtempfänger 36. Bei einem Absinken des Wasserstandes der Verdunsterkammer 20 und damit des Schwimmerelements 24 gelangt das Schwimmerelement 24 aus dem Lichtpfad des Lichtsenders 35 heraus, so dass der von dem Lichtsender 35 abgesendete Lichtstrahl 37 den Lichtempfänger 36 erreicht. Eine Änderung des Signals des Lichtempfängers 36 signalisiert ein Absinken des Wassers unterhalb des Wasserstand-Sollwertes 28 und damit des für den Normalbetrieb der Befeuchtungsvorrichtung 10 kennzeichnenden Wasserstandes.

In einer weiteren Ausführung ist ein Mittel 40 zur Bestimmung der Restzeit des in der Verdunsterkammer 20 verbleibenden Wassers vorgesehen (dargestellt in Figur 1). Aus dem Signal des Füllstandsdetektors 30, einem Signal eines vorzugsweise an dem Atemgasauslasskanal 23 angeordneten Flowsensor 41 (Figur 1) und der Kenntnis des Wasservolumens unterhalb des Wasserstand-Sollwertes 28 der Verdunsterkammer 20 wird in dem Mittel 40 die Restzeit des verbleibenden Wassers der Verdunsterkammer 20 ermittelt. Ferner wird ein entsprechendes Ausgangssignal zur Anzeige und Alarmgabe erzeugt, welches den Anwender auf die verbleibende Restzeit des Wassers in der Verdunsterkammer 20 hinweist.
Alternativ hierzu kann der Flowsensor 41 auch in der Nähe des Ventilators 11 angeordnet sein (nicht dargestellt), wobei das Signal des Flowsensor 41 in dieser Ausführung über eine Datenschnittstelle 18 (Fig. 1) von dem Ventilator 11 der Befeuchtungsvorrichtung 10 zur Verfügung gestellt wird.

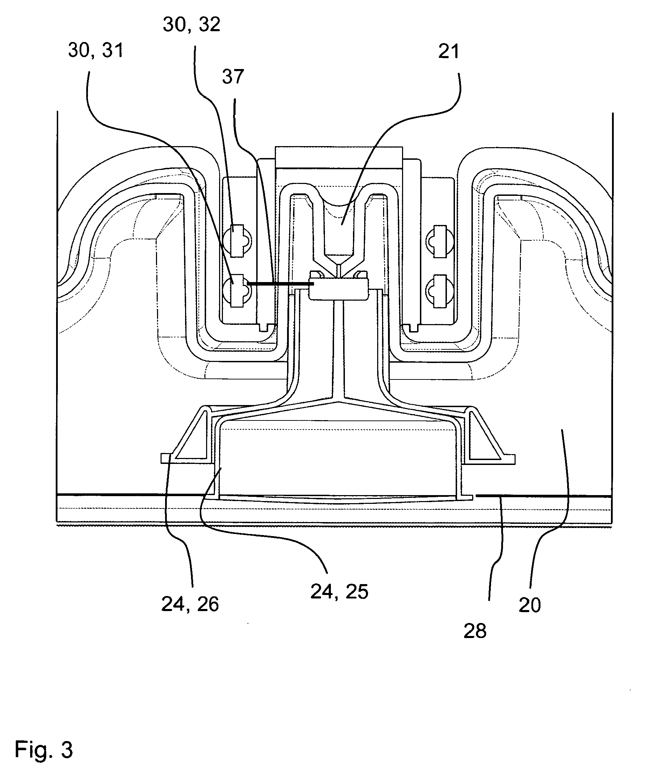

Figur 3 zeigt eine weitere Ausführung der erfindungsgemäßen Befeuchtungsvorrichtung 10, in der ein zweites Schwimmerelement 26 und ein zweiter Füllstandsdetektor 32 in der Verdunsterkammer 20 vorgesehen sind. Die dargestellte Ausführungsform ist dadurch gekennzeichnet, dass das zweite Schwimmerelement 26 das erste Schwimmerelement 25 konzentrisch umgibt und in vorteilhafter Weise das zweite Schwimmerelement 26 auf dem ersten Schwimmerelement 25 aufliegt. Das zweite Schwimmerelement 26 berührt in dieser Position nicht die Wasseroberfläche. Im Fall eines Fehlers der Ventilfunktion des ersten Schwimmerelements 25 in Kombination mit der Wasserzuführung 21 kann der Wasserspiegel in der Verdunsterkammer 20 kontinuierlich ansteigen. Die weitere Wasserzufuhr durch die Wasserzuführung 21 würde nicht gestoppt werden. Ein kritischer Anstieg des Wasserstandes der Verdunsterkammer 20 wäre die Folge. Das auf dem ersten Schwimmerelement 25 aufliegende zweite Schwimmerelement 26 schwimmt in der Ausführung der Figur 3 jedoch mit dem kontinuierlich steigenden Wasserstand der Verdunsterkammer 20 auf. Dabei ist das zweite Schwimmerelement 26 in der Verdunsterkammer über die Höhe des Wasserstand-Sollwertes 28 hinaus bewegbar. Wird das zweite Schwimmerelement 26 über den Wasserstand-Sollwert 28 hinausbewegt, wird ein bisher noch freier Lichtpfad des Lichtsender- und Empfängerpaares des zweiten Füllstandsdetektors 32 durch das zweite Schwimmerelement 26 abgeschattet. In dieser Ausführung sind Mittel vorgesehen, aus dem Signal des zweiten Füllstandsdetektors 32 ein entsprechendes Ausgangssignal zur Anzeige und Alarmgabe dieser Fehlfunktion des ersten Schwimmerelements 25 zu erzeugen (nicht dargestellt).

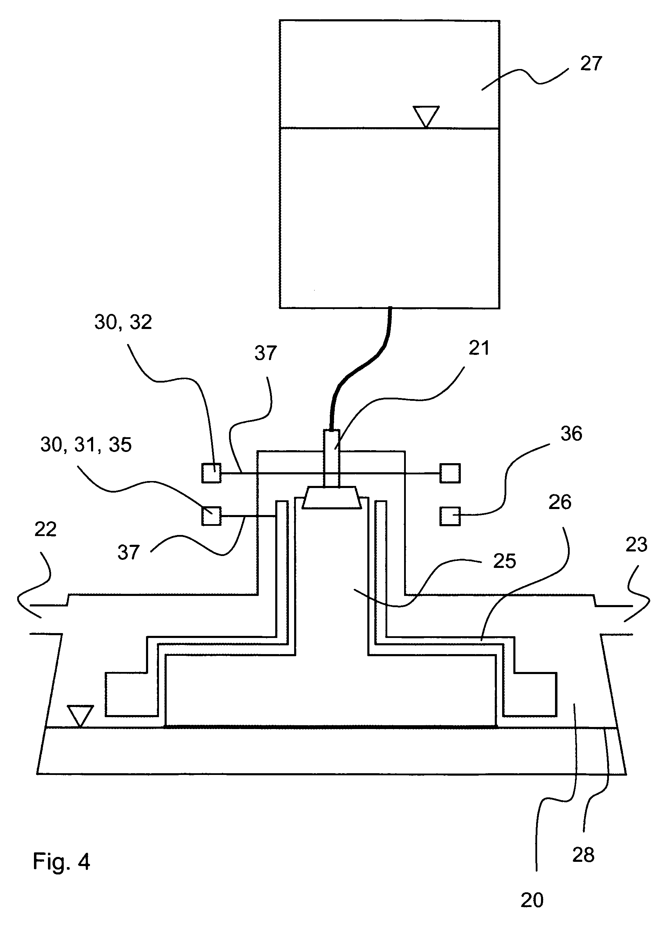

Figur 4 zeigt eine schematische Darstellung der Verdunsterkammer 20 der erfindungsgemäßen Befeuchtungsvorrichtung 10 mit einem Wasserreservoir 27 im Zustand "normaler Betrieb". Die Verdunsterkammer 20 weist in dem Bodenbereich eine Kontaktfläche zur Übertragung von Wärme von der Heizvorrichtung 16 (Fig. 1) auf das Wasser auf. Durch die Wärme der Heizvorrichtung 16 kommt es zu einer Verdunstung des Wassers in der Verdunsterkammer 20. Das erste Schwimmerelement 26 regelt den Wasserstand in der Verdunsterkammer 20 durch Abdichten der Öffnung der Wasserzuführung 21 bei einer Überschreitung des Wasserstandes über dem Wasserstand-Sollwert 28. Die Wasserzuführung 21 ist mit einem separaten Wasserreservoir 27 verbunden. Wenn Wasser benötigt wird, fällt der Wasserstand in der Verdunsterkammer 20, wobei die Wasserzuführung 21 geöffnet wird. Damit kann zusätzliches Wasser aus dem Wasserreservoir 27 in die Verdunsterkammer 20 nachfließen. In der mit Wasser gefüllten Verdunsterkammer 20 schwimmt das erste Schwimmerelement 25 auf der Wasseroberfläche. In einem Anfangszustand einer leeren Verdunsterkammer 20 erfolgt die Wasserzufuhr durch die Wasserzuführung 21 von einem Wasserreservoir 27 solange, bis das erste Schwimmerelement 25 die Wasserzufuhr durch ein Andrücken an die Wasserzuführung 21 unterbricht. In diesem Zustand des normalen Betriebes" wird der Lichtstrahl 37 des Lichtsenders 35 durch das ersten Schwimmerelement 25 unterbrochen, so dass der Lichtstrahl 37 nicht den Lichtempfänger 36 erreicht. Ein von dem zweiten Füllstandsdetektor 32 erzeugter Lichtstrahl 37 wird nicht unterbrochen.

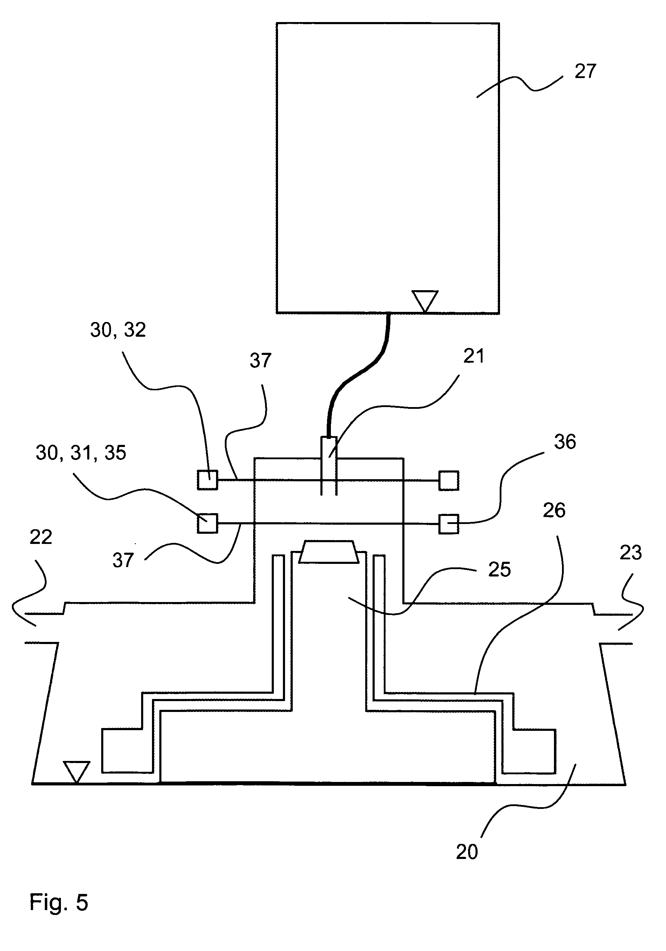

Figur 5 zeigt eine schematische Darstellung der Verdunsterkammer 20 der erfindungsgemäßen Befeuchtungsvorrichtung 10 im Zustand "Wasser leer". Das erste Schwimmerelement 25 befindet sich dabei unterhalb des Wasserstand-Sollwertes 28. Ein von dem Lichtsender 25 ausgesendeter Lichtstrahl 27 der optischen Sensoranordnung 34 des ersten Füllstandsdetektors 31 trifft in dem Zustand "Wasser leer" den Lichtempfänger 36. Zur zeitnahen Erkennung des Zustandes "Wasser leer" der Verdunsterkammer 20, ist ein Mittel 40 vorgesehen in dem aus dem Signal des ersten Füllstandsdetektors 31 und einem Signal eines vorzugsweise an dem Atemgasauslasskanal 23 angeordneten Flowsensor 41 eine Restzeit des verbleibenden Wassers der Verdunsterkammer 20 ermittelt wird (nicht in Fig. 5 dargestellt). Es wird ferner ein entsprechendes Ausgangssignal zur Anzeige und Alarmgabe erzeugt. Damit kann der Anwender zeitnah über eine Restzeit des in der Verdunsterkammer 20 verbleibenden Wasser informiert werden und innerhalb dieser Zeit das Wasserreservoir 27 austauschen bzw. Wasser in das Wasserreservoir 27 auffüllen.

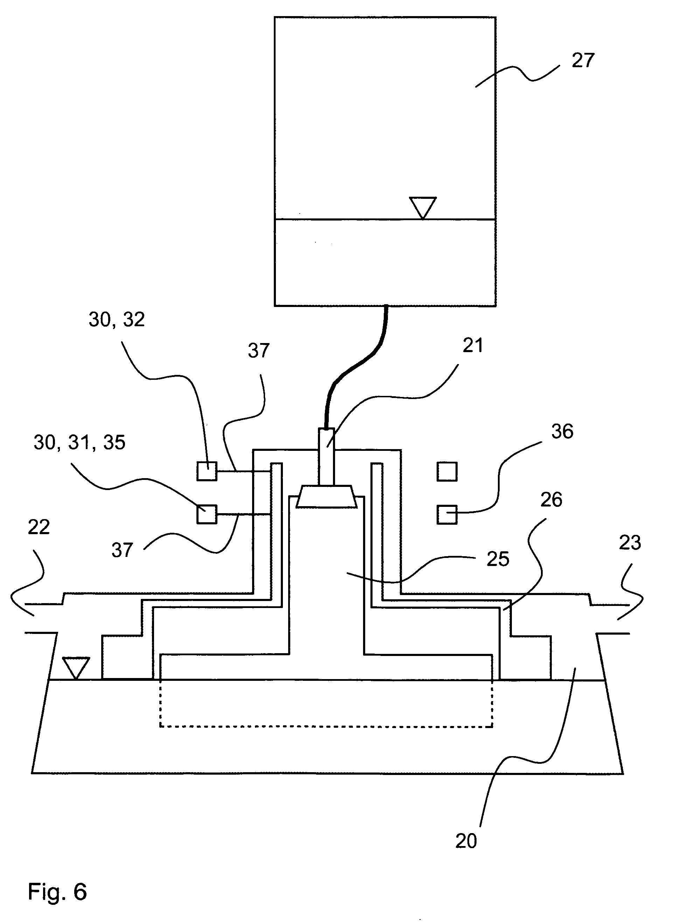

Im Hinblick auf Störungen an dem Schwimmerelementverschluss des ersten Schwimmerelementes 25 ist eine in Fig. 6 dargestellte Ausführung von Bedeutung, die dadurch gekennzeichnet ist, dass das zweites Schwimmerelement 26 und ein zweiter Füllstandsdetektor 32 in der Verdunsterkammer 20 vorgesehen sind, wobei das zweite Schwimmerelement 26 in der Verdunsterkammer 20 über die Höhe des Wasserstand-Sollwertes 28 hinaus bewegbar ist und der zweite Füllstandsdetektor 32 vorgesehen ist, eine Position des zweiten Schwimmerelements 26 oberhalb des Wasserstand-Sollwertes 28 zu erkennen. In Figur 6 ist es somit zu einem Fehlerfall des Zusammenwirkens des ersten Schwimmerelements 25 mit der Wasserzuführung 21 gekommen, so dass der Wasserstand in der Verdunsterkammer 20 kontinuierlich steigt. Dadurch steigt das zweite Schwimmerelement 26 auf und ein Lichtstrahl 37des zweiten Füllstandsdetektors 32 wird unterbrochen. In Abhängigkeit von der Position des zweiten Schwimmerelementes 26 kann somit ein kritisches Ansteigen des Wassers in der Verdunsterkammer 20 oberhalb des Wasserstand-Sollwertes 28 erkannt werden. Ferner sind in vorteilhafter Weise Mittel vorgesehen, aus dem Signal des zweiten Füllstandsdetektors 32 ein entsprechendes Ausgangssignal zur Anzeige und Alarmgabe zu erzeugen (nicht dargestellt).

Sowohl der erste als auch der zweite Füllstandsdetektor 31 und 32 kann als eine magnetische Sensoranordnung ausgeführt sein, die aus einem Hallsensor und ein an dem jeweiligen Schwimmerelement einstückig angeformten Magnet besteht (nicht dargestellt).

Während die vorliegende Erfindung unter Bezugnahme auf die bevorzugten Ausführungsbeispiele beschrieben worden ist, sind dem Fachmann verschiedene Änderungen und Modifikationen klar. All diese Änderungen und Modifikationen sollen in den Schutzbereich der angefügten Ansprüche fallen.

### BEZUGSZEICHENLISTE

- 10: Befeuchtungsvorrichtung
- 11: Ventilator
- 12: Gehäuse
- 14: Ausnehmung
- 16: Heizvorrichtung
- 18: Datenschnittstelle

- 20: Verdunsterkammer
- 21: Wasserzuführung
- 22: Atemgaszuführungskanal
- 23: Atemgasauslasskanal
- 24: Schwimmerelement
- 25: erstes Schwimmerelement
- 26: zweites Schwimmerelement
- 27: Wasserreservoir
- 28: Wasserstand-Sollwert

- 30: Füllstandsdetektor
- 31: erster Füllstandsdetektor
- 32: zweiter Füllstandsdetektor
- 34: optische Sensoranordnung
- 35: Lichtsender
- 36: Lichtempfänger
- 37: Lichtstrahl

- 40: Mittel zur Bestimmung der Restzeit
- 41: Flowsensor

## Patentansprüche

1. Befeuchtungsvorrichtung (10) für Atemgase,
umfassend ein Gehäuse (12) mit einer Ausnehmung (14),
eine Heizvorrichtung (16),
eine Verdunsterkammer (20) mit einer Wasserzuführung (21), einen Atemgaszuführungskanal (22), einen Atemgasauslasskanal (23) und wenigstens ein Schwimmerelement (24, 25, 26),
wobei die Verdunsterkammer (20) in die Ausnehmung (14) wenigstens teilweise einbringbar ist, in Kontakt mit der Heizvorrichtung (16) zum Erwärmen von Wasser steht und ausgeführt ist, Wasser aus einem Wasserreservoir (27) durch die Wasserzuführung (21) aufzunehmen,
wobei das wenigstens eine Schwimmerelement (24, 25, 26) auf dem Wasser schwimmend beweglich geführt ist und ausgebildet ist, die Wasserzuführung (21) oberhalb eines Wasserstand-Sollwertes (28) in der Verdunsterkammer (20) zu verschließen
**dadurch gekennzeichnet, dass**
wenigstens ein Füllstandsdetektor (30, 31, 32) vorgesehen ist, der ausgebildet ist, in Abhängigkeit von der Position des wenigstens einen Schwimmerelements (24, 25, 26) ein Absinken des Wassers von dem Wasserstand-Sollwert (28) zu erkennen.

2. Vorrichtung nach Anspruch 1, **dadurch gekennzeichnet, dass** die Verdunsterkammer (20) einstückig aus einem lichtdurchlässigen Material, vorzugsweise aus einem PU, ausgeführt ist.

3. Vorrichtung nach einem der vorhergehenden Ansprüche, **dadurch gekennzeichnet, dass** der wenigstens eine Füllstandsdetektor (30, 31, 32) als eine auf den Wasserstand-Sollwert (28) angepasste optische Sensoranordnung (34) ausgeführt ist, bestehend aus einem Lichtsender (35) und einem Lichtempfänger (36).

4. Vorrichtung nach Anspruch 3, **dadurch gekennzeichnet, dass** die optische Sensoranordnung (34) außerhalb der Verdunsterkammer (20) angeordnet ist und so eingestellt ist, dass ein von dem Lichtsender (35) in die Verdunsterkammer (20) ausgesendeter Lichtstrahl (37) von dem wenigstens einen Schwimmerelement (24, 25, 26) unterbrochen wird, wenn die Höhe des Wassers in der Verdunsterkammer (20) den Wasserstand-Sollwert (28) erreicht.

5. Vorrichtung nach Anspruch 1, **dadurch gekennzeichnet, dass** der wenigstens eine Füllstandsdetektor (30, 31, 32) als eine auf den Wasserstand-Sollwert (28) angepasste magnetische Sensoranordnung ausgeführt ist, bestehend aus einem Hallsensor und einem an dem wenigstens einen Schwimmerelement (24, 25, 26) einstückig angeformten Magneten.

6. Vorrichtung nach einem der vorhergehenden Ansprüche, **dadurch gekennzeichnet, dass** Mittel (40) vorgesehen sind, aus dem Signal des wenigstens einen Füllstandsdetektors (30, 31, 32) und einem Signal eines Flowsensors (41) eine Restzeit des in der Verdunsterkammer (20) verbleibenden Wassers zu ermitteln und ein entsprechendes Ausgangssignal zur Anzeige und Alarmgabe zu erzeugen.

7. Vorrichtung nach einem der vorhergehenden Ansprüche, **dadurch gekennzeichnet, dass** ein zweites Schwimmerelement (24, 26) und ein zweiter Füllstandsdetektor (30, 32) in der Verdunsterkammer (20) vorgesehen sind, wobei das zweite Schwimmerelement (24, 26) in der Verdunsterkammer (20) über die Höhe des Wasserstand-Sollwert (28) hinaus bewegbar ist und der zweite Füllstandsdetektor (30, 32) vorgesehen ist, eine Position des zweiten Schwimmerelements (24, 26) oberhalb des Wasserstand-Sollwert (28) zu erkennen.

8. Vorrichtung nach Anspruch 7, **dadurch gekennzeichnet, dass** Mittel (40) vorgesehen sind, aus dem Signal des zweiten Füllstandsdetektors (30, 32) ein entsprechendes Ausgangssignal zur Anzeige und Alarmgabe zu erzeugen.

9. Vorrichtung nach einem der vorhergehenden Ansprüche, **dadurch gekennzeichnet, dass** das zweite Schwimmerelement (24, 26) das erste Schwimmerelement (24, 25) konzentrisch umgibt.

10. Vorrichtung nach Anspruch 9, **dadurch gekennzeichnet, dass** das zweite Schwimmerelement (24, 26) auf dem ersten Schwimmerelement (24, 25) aufliegt.

11. Verfahren zum Betreiben einer Befeuchtungsvorrichtung (10) für Atemgase, mit den folgenden Verfahrensschritten:
Zuführung von Wasser von einem Wasserreservoir (27) in eine Verdunsterkammer (20),
Sperren der Wasserzuführung (21) der Verdunsterkammer (20) durch einen Schwimmerelement (24) bei Überschreitung eines Wasserstandssollwertes (28) in der Verdunsterkammer (20),
Erwärmung des Wassers in der Verdunsterkammer (20) durch eine Heizvorrichtung (16)
**dadurch gekennzeichnet, dass**
ein Absinken des Wassers von einem Wasserstand-Sollwerte (28) mit einem Füllstandsdetektor (30) in Abhängigkeit von der Position des Schwimmerelements (24) detektiert wird.

12. Verfahren nach Anspruch 11, **dadurch gekennzeichnet, dass** jeweils ein Signal des Füllstandsdetektors (30) und ein Signal eines vorzugsweise an einem Atemgasauslasskanal (23) angeordneten Flowsensors (41) erfasst werden.

13. Verfahren nach Anspruch 12, **dadurch gekennzeichnet, dass** aus den Signalen nach dem Verfahrensschritt d) eine Restzeit des in der Verdunsterkammer (20) verbleibenden Wassers bestimmt wird.

14. Verfahren nach Anspruch 13, **dadurch gekennzeichnet, dass** in Abhängigkeit der Restzeit des in der Verdunsterkammer (20) verbleibenden Wassers ein entsprechendes Ausgangssignal zur Anzeige und Alarmgabe erzeugt wird.

## Claims

1. Humidifying device (10) for breathing gases, comprising a housing (12) with a recess (14), a heater (16), an evaporating chamber (20) with a water feed (21), a breathing gas feed channel (22), a breathing gas outlet channel (23) and at least one float element (24, 25, 26), said evaporating chamber (20) being introducible, at least in part, into said recess (14), being in contact with said heater (16) for heating water and being configured to receive water from a water reservoir (27) through said water feed (21), said at least one float element (24, 25, 26) being guided in a floatingly movable manner on the water and configured to close the water feed (21) above a water level setpoint (28) in said evaporating chamber (20), **characterised in that** at least one filling level detector (30, 31, 32) is provided which is configured to recognise a fall in the water from the water level setpoint (28) as a function of the position of said at least one float element (24, 25, 26):

2. Device according to claim 1, **characterised in that** the evaporating chamber (20) is formed in one piece from a transparent material, preferably from a PU.

3. Device according to either of the preceding claims, **characterised in that** said at least one filling level detector (30, 31, 32) is formed as optical sensor array (34) adapted to the water level setpoint (28) and consisting of a light transmitter (35) and a light receiver (36).

4. Device according to claim 3, **characterised in that** the optical sensor array (34) is arranged outside the evaporating chamber (20) and is set in such a way that a light beam (37) emitted by said light transmitter (35) into said evaporating chamber (20) is interrupted by said at least one float element (24, 25, 26) when the level of the water in the evaporating chamber (20) reaches the water level setpoint (28).

5. Device according to claim 1, **characterised in that** said at least one filling level detector (30, 31, 32) is configured as a magnetic sensor array adapted to the water level setpoint (28) and consisting of a Hall sensor and a magnet formed integrally in one piece with said at least one float element (24, 25, 26).

6. Device according to any one of the preceding claims, **characterised in that** means (40) are provided for detecting, from the signal of said at least one filling level detector (30, 31, 32) and a signal of a flow sensor (41), a remaining time during which the water will still be present in said evaporating chamber (20) and for generating a corresponding output signal for display and for triggering an alarm.

7. Device according to any one of the preceding claims, **characterised in that** a second float element (24, 26) and a second filling level detector (30, 32) are provided in said evaporating chamber (20), said second float element (24, 26) being movable in said evaporating chamber (20) above the level of the water level setpoint (28) and said second filling level detector (30, 32) being provided to recognise a position of said second float element (24, 26) above said water level setpoint (28).

8. Device according to claim 7, **characterised in that** means (40) are provided for generating, from the signal of said second filling level detector (30, 32), a corresponding output signal for display and for triggering an alarm.

9. Device according to any one of the preceding claims, **characterised in that** said second float element (24, 26) concentrically surrounds said first float element (24, 25).

10. Device according to claim 9, **characterised in that** said second float element (24, 26) lies on said first float element (24, 25).

11. Process for operating a humidifying device (10) for breathing gases, the process comprising the steps of:
feeding water from a water reservoir (27) into an evaporating chamber (20),
blocking the water feed (21) to the evaporating chamber (20) by means of a float element (24) when a water level setpoint (28) is exceeded in the evaporating chamber (20),
heating the water in the evaporating chamber (20) by means of a heater (16),
**characterised in that** a fall in the water from a water level setpoint (28) is detected by a filling level detector (30) as a function of the position of the float element (24).

12. Process according to claim 11, **characterised in that** a signal of the filling level detector (30) and a signal of a flow sensor (41), preferably arranged at a breathing gas outlet channel (23), are each detected.

13. Process according to claim 12, **characterised in that** a remaining time during which water will still be present in the evaporating chamber (20) is determined from the signals detected in accordance with method step d).

14. Process according to claim 13, **characterised in that** a corresponding output signal is generated, as a function of the remaining time during which water will still be present in the evaporating chamber (20), for display and for triggering an alarm.

## Revendications

1. Dispositif d'humidification (10) pour des gaz respiratoires, qui comprend :
- un boîtier (12) avec une cavité (14),
- un dispositif de chauffage (16),
- une chambre d'évaporation (20) avec une amenée d'eau (21), un canal d'amenée de gaz respiratoire (22), un canal de sortie de gaz respiratoire (23) et au moins un élément flottant (24, 25, 26), étant précisé que la chambre d'évaporation (20) peut être engagée au moins en partie dans la cavité (14), est en contact avec le dispositif de chauffage (16) pour chauffer l'eau et est conçue pour accueillir l'eau provenant d'un réservoir d'eau (27) par l'intermédiaire d'une amenée d'eau (21), que l'élément flottant (24, 25, 26) au nombre d'un au moins peut se déplacer sur l'eau et qu'il est constitué de manière à fermer l'amenée d'eau (21) quand est dépassée une valeur de consigne de niveau d'eau (28) dans la chambre d'évaporation,
ce dispositif d'humidification étant **caractérisé en ce qu'**il est prévu au moins un détecteur de remplissage (30, 31, 32) constitué pour, en fonction de la position de l'élément flottant (24, 25, 26) au nombre d'un au moins, détecter un abaissement de l'eau en dessous de la valeur de consigne de niveau d'eau (28).

2. Dispositif selon la revendication 1, **caractérisé en ce que** la chambre de vaporisation (20) est réalisée monobloc en un matériau transparent à la lumière, de préférence un PU.

3. Dispositif selon une des revendications précédentes, **caractérisé en ce que** le détecteur de remplissage (30, 31, 32), au nombre d'un au moins est constitué sous la forme d'un dispositif capteur (34) optique ajusté à la valeur de consigne de niveau d'eau (28) et qui est composé d'un émetteur de lumière (35) et d'un récepteur de lumière (36).

4. Dispositif selon la revendication 3, **caractérisé en ce que** le dispositif capteur (34) optique est disposé en dehors de la chambre de vaporisation (20) et qu'il est réglé de manière qu'un rayon lumineux (37) envoyé par l'émetteur de lumière (35) dans la chambre d'évaporation (20) est interrompu par l'élément flottant (24, 25, 26) au nombre d'un au moins quand le niveau de l'eau dans la chambre d'évaporation (20) atteint la valeur de consigne (28).

5. Dispositif selon la revendication 1, **caractérisé en ce que** le détecteur de remplissage (30, 31, 32) au nombre d'un au moins est un dispositif capteur magnétique ajusté à la valeur de consigne de niveau d'eau (28) et qui est composé d'un capteur à effet Hall et d'un aimant réalisé monobloc sur l'élément flottant (24, 25, 26).

6. Dispositif selon une des revendications précédentes, **caractérisé en ce que** des moyens sont prévus pour établir, à partir du signal d'un détecteur de remplissage (30, 31, 32) au nombre d'un au moins et d'un signal d'un capteur de flux (41) le temps résiduel de présence de l'eau qui se trouve dans la chambre de vaporisation (20) et de délivrer un signal de sortie correspondant, pour informer et donner l'alarme.

7. Dispositif selon une des revendications précédentes, **caractérisé en ce qu'**un second élément flottant (24, 26) et un second détecteur de remplissage (30, 31) sont prévus dans la chambre de vaporisation (20), que ce second élément flottant (24, 26) peut se déplacer au-delà de la hauteur de la valeur de consigne de niveau d'eau (28), le second détecteur de remplissage (30, 32) étant prévu pour détecter une position du second élément flottant (24, 26) au-dessus de la valeur de consigne de niveau d'eau (28).

8. Dispositif selon la revendication 7, **caractérisé en ce que** des moyens (40) sont prévus pour délivrer, à partir d'un signal émis par le second détecteur de remplissage (30, 32), un signal de sortie correspondant qui sert à informer et à donner l'alarme.

9. Dispositif selon une des revendications précédentes, **caractérisé en ce que** le second élément flottant (24, 26) entoure concentriquement le premier élément flottant (24, 25).

10. Dispositif selon la revendication 9, **caractérisé en ce que** le second élément flottant (24, 26) repose sur le premier élément flottant (24, 25).

11. Procédé pour exploiter un dispositif d'humidification (10) de gaz respiratoires, qui comporte les étapes suivantes :
- à partir d'un réservoir d'eau (27) amener de l'eau à une chambre d'évaporation,
- couper l'amenée d'eau (21) à la chambre d'évaporation (20) au moyen d'un élément flottant (24) quand est dépassée dans la chambre d'évaporation (20) une valeur de consigne de niveau d'eau (28),
- chauffer l'eau dans la chambre d'évaporation (20) au moyen d'un dispositif de chauffage (16),
ce procédé étant **caractérisé en ce qu'**un abaissement du niveau de l'eau par rapport à une valeur de consigne (28) de ce niveau est détecté en fonction de la position de l'élément flottant (24).

12. Procédé selon la revendication 11, **caractérisé en ce que** chaque fois sont saisis un signal du détecteur de remplissage (30) et un signal d'un détecteur de flux (41) disposé de préférence sur un canal de sortie de gaz respiratoire (23).

13. Procédé selon la revendication 12, **caractérisé en ce qu'**après l'étape d) du procédé, est déterminé le temps résiduel de présence de l'eau qui se trouve dans la chambre d'évaporation (20).

14. Procédé selon la revendication 13, **caractérisé en ce qu'**en fonction du temps résiduel de présence de l'eau qui se trouve dans la chambre d'évaporation (20) est produit un signal de sortie qui sert à informer et à donner l'alarme.
